# FASCICULE DE BREVET EUROPEEN

(11) **EP 1 523 359 B1**
(45) Date de publication et mention de la délivrance du brevet: **26.06.2013**
(21) Numéro de dépôt: 03748215.5
(22) Date de dépôt: 16.07.2003
(51) Int. Cl.: A61M 5/32, A61M 5/24

(54) **DISPOSITIF D'INJECTION D'UN PRODUIT, NOTAMMENT A USAGE MEDICAL**
INJEKTIONS- ODER ZERSTÄUBUNGSVORRICHTUNG, INSBESONDERE ZUM MEDIZINISCHEN GEBRAUCH
DEVICE FOR INJECTING A PRODUCT, IN PARTICULAR FOR MEDICAL USE

(30) Priorité: 19.07.2002 FR 0209238
(43) Date de publication de la demande: 20.04.2005
(73) Titulaire: Becton Dickinson France, 38800 Le Pont de Claix (FR)
(72) Inventeur: VEDRINE, Lionel, Palo Alto, California 94306 (US)
(74) Mandataire: Brédeville, Odile Marie
(86) Numéro de dépôt international: PCT/FR2003/002252
(87) Numéro de publication internationale: WO 2004/009164

(56) Documents cités:
- WO-A-01/45776
- WO-A-98/13077
- WO-A-99/47194
- FR-A- 2 750 051
- US-A- 5 320 606

## Description

La présente invention concerne un dispositif d'injection d'un produit, notamment à usage médical. Ce dispositif est notamment destiné à permettre de réaliser une injection intra-dermale.

Dans la description ci-après, les termes "proximal" et "distal" sont considérés par rapport au sens d'injection du produit.

Il est courant de réaliser une injection intra-dermale au moyen d'une seringue classique, en piquant selon une direction formant un angle faible avec la peau.

Le document WO 01/45776 décrit une seringue dont l'aiguille peut être rétractée à l'intérieur du corps de la seringue après l'injection.

Ces seringues classiques n'assurent pas une parfaite fiabilité de l'injection ni une parfaite sécurité contre les risques de piqûres accidentelles susceptibles de se produire après l'injection.

L'invention vise à remédier à cet inconvénient fondamental.

L'objectif de l'invention est donc de fournir un dispositif assurant une parfaite fiabilité de injection et une parfaite sécurité contre les risques de piqûres accidentelles.

Cet objectif est atteint par un dispositif selon la revendication 1.

Lors de la réalisation de l'injection, l'aiguille est maintenue par rapport au corps en position d'injection et le récipient est maintenu par rapport au poussoir en position d'injection. Le déplacement du poussoir par rapport au corps dans le sens du rapprochement de l'extrémité fermée du récipient et de l'aiguille, amène le piston dans ladite deuxième conformation ou position, permettant le passage du produit vers l'extérieur du récipient.

En fin d'injection, lesdits moyens d'actionnement provoquent respectivement le relâchement desdits moyens de maintien de l'aiguille et desdits moyens de maintien du récipient, ce qui permet d'amener l'aiguille et le récipient en position de rétraction. Cette rétraction permet d'assurer une parfaite sécurité contre les risques de piqûres accidentelles.

Le piston peut être conformé pour, dans ladite deuxième conformation ou position, permettre le passage du produit entre lui et le récipient. Le piston peut notamment comprendre au moins une zone périphérique, propre, dans ladite première conformation du piston, à appuyer étroitement contre la paroi du récipient, et, dans ladite deuxième conformation du piston, à s'effacer sous la pression du produit à injecter pour permettre le passage de ce dernier.

Le piston peut également comprendre une zone transperçable placée en regard de l'extrémité proximale de l'aiguille. Le déplacement du récipient par rapport à l'aiguille conduit alors l'extrémité proximale de l'aiguille à transpercer cette zone transperçable du piston jusqu'à venir en communication avec le produit à injecter et permettre l'écoulement de ce produit à travers l'aiguille.

Avantageusement, le dispositif comprend des moyens à ressort permettant d'amener l'aiguille et le récipient en position de rétraction sans intervention volontaire extérieure.

Avantageusement, ledit corps forme une paroi distale perpendiculaire à l'axe de l'aiguille, de laquelle l'aiguille dépasse, en position d'injection, sur une longueur correspondant à la profondeur d'enfoncement recherchée de cette aiguille lors de l'injection.

Cette paroi distale forme une paroi d'arrêt, destinée à venir en appui contre la peau lors de d'enfoncement de l'aiguille afin de limiter cet enfoncement à ladite profondeur recherchée.

Le dispositif selon l'invention est ainsi particulièrement adapté à la réalisation d'injections intra-dermales.

En position de rétraction, l'aiguille peut simplement être rétractée en deçà de cette paroi, du côté proximal, pour éliminer le risque de piqûre accidentelle après injection.

Selon une possibilité de mise en oeuvre de l'invention, lesdits moyens de maintien de l'aiguille comprennent :
- une pièce supportant l'aiguille, comportant au moins un moyen de verrouillage ;
- au moins une patte comportant un moyen de verrouillage propre à coopérer avec celui de ladite pièce supportant l'aiguille, cette patte étant mobile radialement entre une position radialement interne normale, dans laquelle lesdits moyens de verrouillage viennent en prise de manière à maintenir ladite pièce supportant l'aiguille par rapport audit corps, et une position radialement externe, dans laquelle une zone du poussoir vient déplacer cette patte radialement vers l'extérieur de manière à libérer ledit verrouillage, ce qui libère par conséquent ladite pièce supportant l'aiguille par rapport audit corps.

Selon une possibilité de mise en oeuvre de l'invention, lesdits moyens de maintien du récipient comprennent :
- une collerette formée au niveau de l'extrémité du récipient opposée à l'extrémité fermée de ce récipient ;
- des moyens de prise solidaires dudit poussoir, permettant de relier ladite collerette au poussoir ; et
- au moins une patte comportant lesdits moyens de prise, mobile dans le sens radial de ce poussoir, entre une position radialement interne, dans laquelle lesdits moyens de prise relient ladite collerette au poussoir, et une position radialement externe, dans laquelle lesdits moyens de prise sont effacés radialement au delà cette collerette, qu'ils libèrent par conséquent.

Les figures annexées illustrent, à titre d'exemple, un mode de réalisation préféré du dispositif selon l'invention.
La figure 1 en est une vue en perspective ;
la figure 2 en est une vue en perspective éclatée, en coupe passant par son axe ;
la figure 3 en est une vue en perspective et en coupe passant par son axe, à l'état monté ;
la figure 4 est une vue partielle, à échelle agrandie, de son extrémité distale, en coupe selon un plan perpendiculaire au plan de coupe des figures 2 et 3, dans une position de maintien de moyens de maintien de l'aiguille que comprend ce dispositif ;
la figure 5 est une vue du dispositif similaire à la figure 4, dans une position de relâchement de ces moyens de maintien de l'aiguille ;
les figures 6 à 8 en sont des vues partielles, à échelle agrandie, en coupe passant par son axe, respectivement en position de stockage, en cours d'injection, et en fin d'injection, et
la figure 9 montre, à échelle agrandie, un capuchon que comprend le dispositif.

Les figures représentent un dispositif 1 d'injection d'un produit, notamment à usage médical.

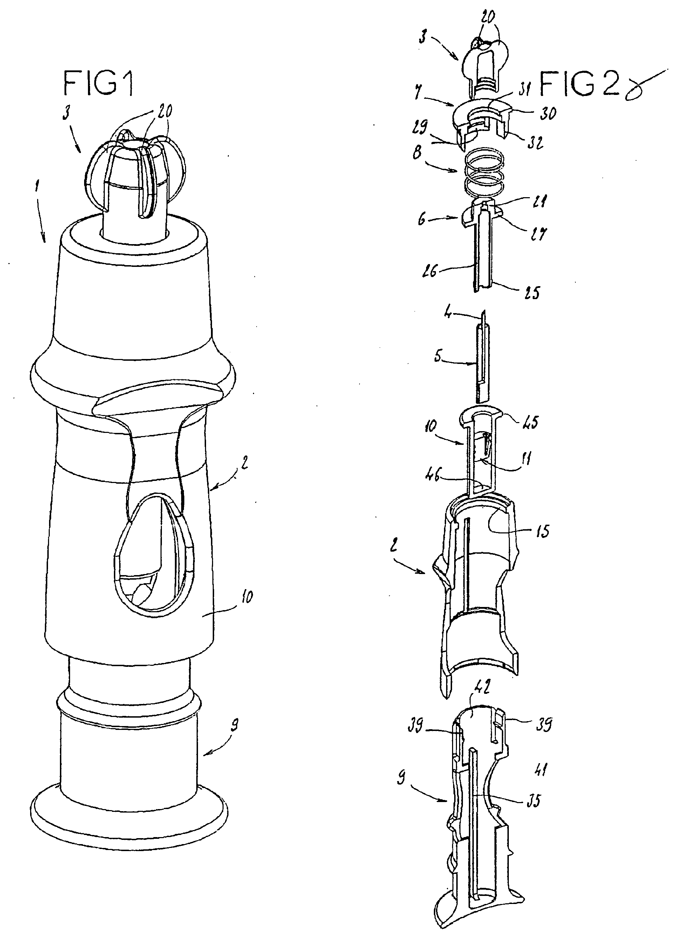

Comme le montre plus particulièrement la figure 2, le dispositif 1 comprend un corps 2, un capuchon distal de protection 3, une aiguille creuse d'injection 4, des pièces 5 à 7 de montage de l'aiguille 4, un ressort 8, un poussoir 9, un récipient 10 et un piston 11, décrits en détails ci-après.

Le corps 2 présente une forme générale tubulaire, et comprend une nervure circulaire 15 au niveau de son extrémité distale.

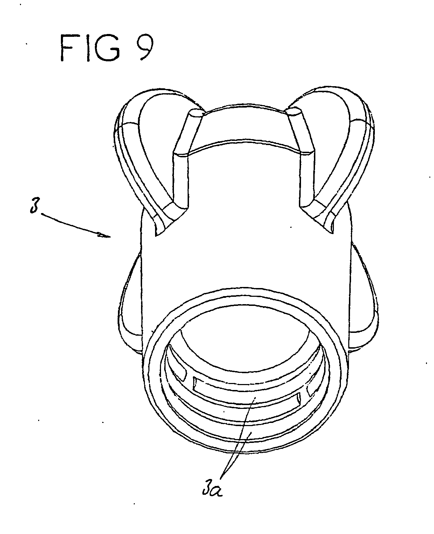

Le capuchon 3 présente des ailes de préhension 20 et peut être clipé sur un bossage 21 que forme l'une des pièces 6 de montage de l'aiguille 4. Comme le montre la figure 9, ce capuchon 3 peut présenter au moins une ouverture pour permettre l'échappement de l'air lors de l'insertion d'une partie 26 de la pièce 6 dans le récipient 10, comme cela apparaîtra plus loin. Dans la forme de réalisation représentée sur cette figure 9, le capuchon 3 présente plusieurs nervures interrompues 3a, étagées dans le sens axial, dont les interruptions sont décalées angulairement pour former un labyrinthe.

L'aiguille 4 est fixée à la pièce 5. Celle-ci a une forme générale cylindrique et présente une rainure et un perçage qui forment un conduit d'écoulement communiquant avec la cavité de l'aiguille 4.

La pièce 6 présente une partie proximale 26 de forme tubulaire qui reçoit étroitement la pièce 5 en elle, et comprend un trou distal pour permettre l'engagement de l'aiguille 4 au travers du bossage 21. La partie 26 est destinée à être introduite dans le récipient 10, comme mentionné plus haut et comporte un joint d'étanchéité 25 à son niveau proximal. Cette partie 26 permet ainsi de déplacer le piston 11 dans le récipient 10 lorsque le poussoir 9 est déplacé par rapport au corps 2, comme cela apparaîtra plus loin.

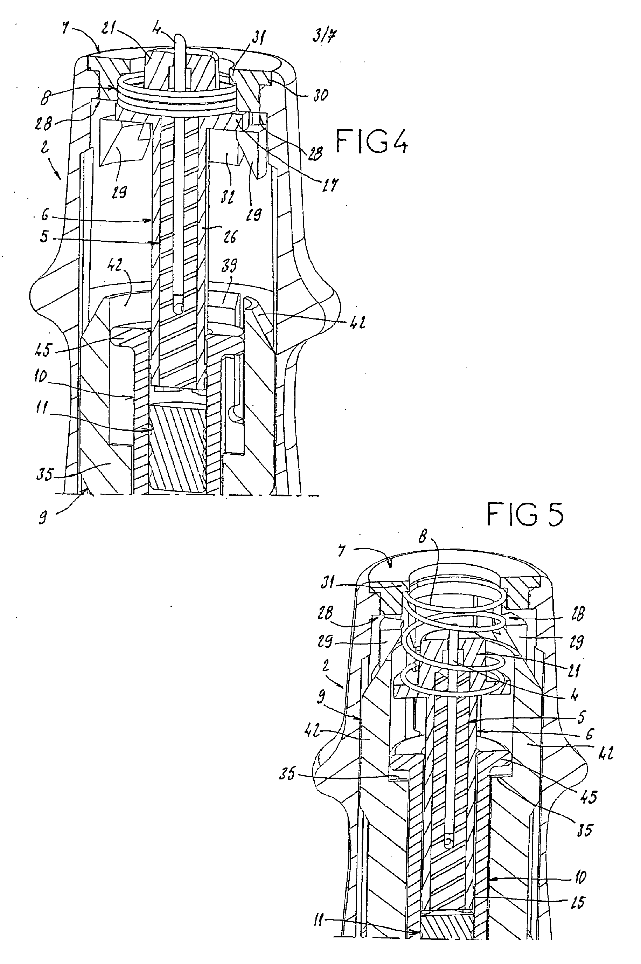

La pièce 6 comprend également une collerette 27 propre à être clipée dans des ouvertures 28, plus particulièrement visibles sur la figure 4, que présentent deux pattes 29 solidaires de la pièce 7, mobiles radialement par rapport à celle-ci.

La pièce 7 est destinée à être insérée étroitement dans l'ouverture délimitée par la nervure distale 15 du corps 2, une collerette distale 30 qu'elle comprend prenant place dans l'évidement distal délimité par cette nervure 15. Cet engagement étroit permet la fixation de la pièce 7 au corps 2.

La pièce 7 comprend également une ouverture permettant le passage du bossage 21 et la mise en place du capuchon 3 sur ce bossage. Cette ouverture est délimitée par un rebord 31 de diamètre inférieur au diamètre du ressort 8.

Comme le montre la figure 4, ce rebord 31 permet le maintien du ressort 8 à l'état comprimé entre la face proximale de ce rebord 31 et la face distale de la collerette 27 lorsque la pièce 6 est clipée à la pièce 7.

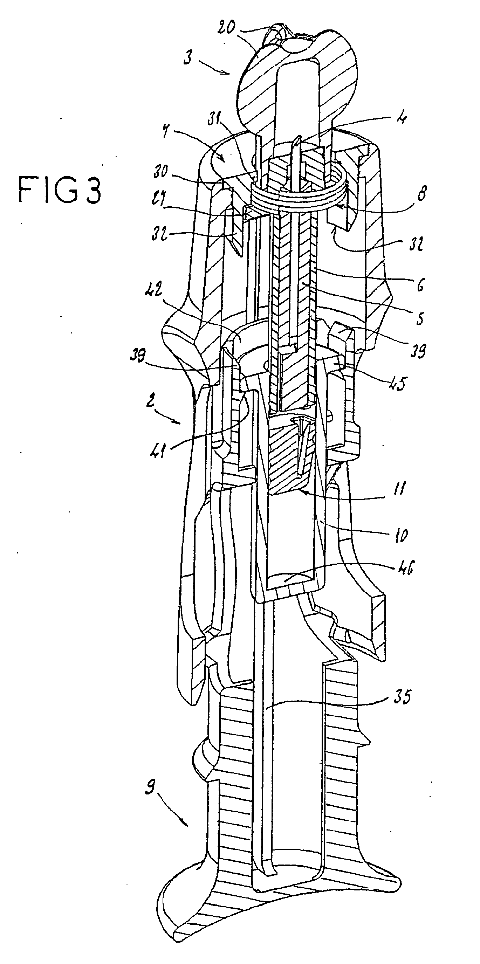

La pièce 7 comprend en outre des parois 32 entre les pattes 29. Il apparaît sur les figures 3 et 4 que les pattes 29 présentent des rampes inclinées internes aménagées dans leurs parties proximales tandis que les parois 32 présentent des rampes inclinées externes aménagées dans leurs parties proximales.

Le poussoir 9 est engagé dans le corps 2 et peut coulisser par rapport à celui-ci. Il comprend des nervures latérales 35 de guidage latéral du récipient 10.

À son niveau proximal, le poussoir 9 forme deux pattes 39 mobiles radialement, pourvues de saillies internes 41 formant des butées de réception d'une collerette 45 que comprend le récipient 10. Cette venue en butée de la collerette 45 contre les saillies 41 permet de lier la collerette 45 au poussoir 9 dans le sens de déplacement du poussoir 9 qui permet de réaliser l'injection.

Le poussoir 9 forme également deux parois 42 situées entre les pattes 39. Comme le montrent les figures 3 et 4, les pattes 39 comprennent, au niveau de leurs extrémités distales, des rampes inclinées internes propres à venir coopérer avec les rampes des parois 32 en fin de course d'injection, et les parois 42 comprennent, au niveau de leurs extrémités distales, des rampes inclinées externes propres à venir coopérer avec les rampes internes des pattes 29 également en fin de course d'injection.

Du côté opposé à la collerette 45, le récipient 10 comprend un fond 46. Le produit à injecter est contenu entre le piston 11 et les parois du récipient 10.

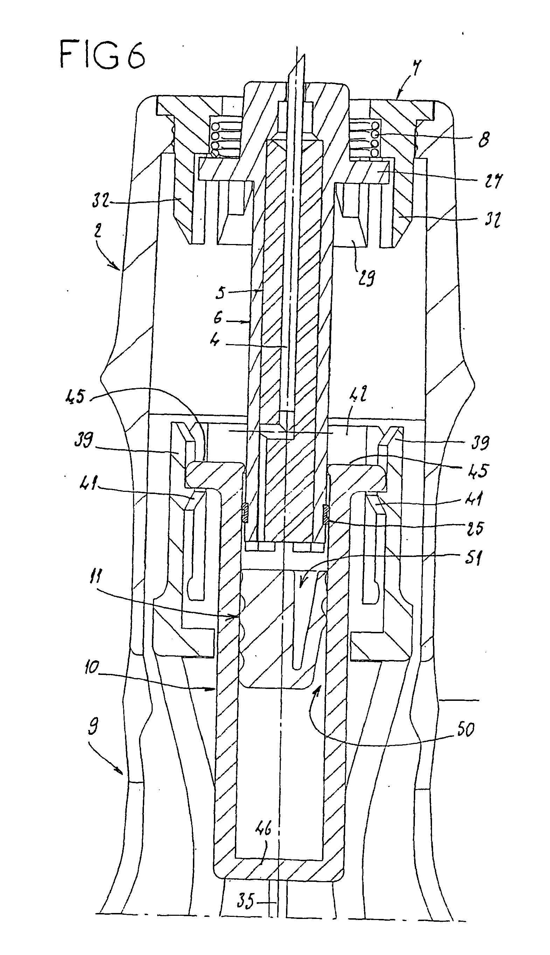

Le piston 11 est en une matière souple, notamment en élastomère. Il présente une forme conique et est placé dans le récipient 10 de telle sorte que sa face de plus faible surface soit tournée vers le produit à injecter. Il ménage ainsi, comme le montre la figure 6, un interstice 50 entre lui et la paroi du récipient 10. De plus, le piston 11 comprend un trou borgne latéral 51 aménagé sur une majeure partie de son épaisseur, à partir de sa face axiale distale, du côté de la paroi latérale du piston 11 qui permet de délimiter ledit interstice 50. Le trou 51 a une forme telle qu'il suit, au moins approximativement, cette paroi latérale, et délimite ainsi une zone périphérique s'étendant sur une portion de la périphérie du piston 11.

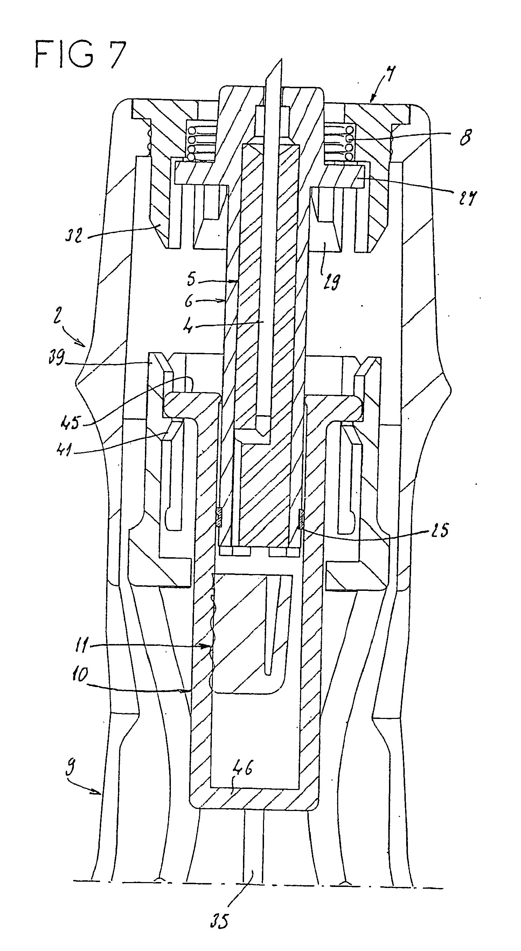

Comme le montre la comparaison des figures 6 et 7, cette zone périphérique adopte normalement une position radialement externe montrée sur la figure 6, dans laquelle elle appuie étroitement contre la paroi du récipient 10, et peut prendre une position radialement interne montrée sur la figure 7, dans laquelle elle s'efface sous la pression du produit à injecter lors du passage de ce dernier entre le piston 11 et le récipient 10, résultant de l'appui du piston 11 contre le produit.

En pratique, le dispositif 1 se trouve à l'origine dans la position de stockage représentée sur la figure 6, dans laquelle la collerette 27 est en prise avec les pattes 29 et la collerette 45 est maintenue par les saillies 41. Dans cette position, l'aiguille 4 fait saillie au-delà de l'extrémité distale du dispositif selon la profondeur recherchée pour l'injection, qui est une injection intra-dermale dans l'exemple représenté.

Le déplacement du récipient 10 avec le poussoir 9 presse le piston 11 contre le produit à injecter, ce qui amène à l'écoulement du produit entre le piston 11 et le récipient 10, comme cela apparaît sur la figure 7. L'injection est alors réalisée en poursuivant le déplacement du poussoir 9 par rapport au corps 2.

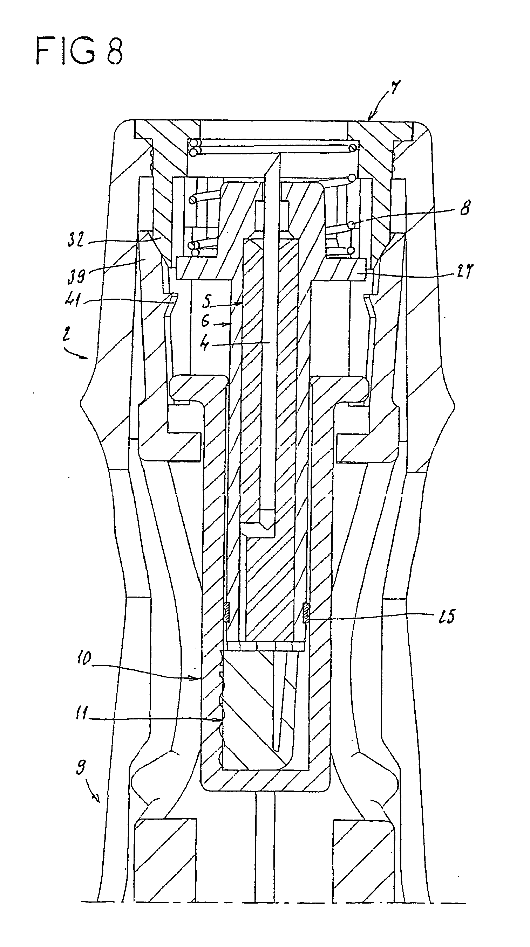

À l'approche de la position de fin de course d'injection, les rampes des pattes 39 et des parois 42 viennent porter contre, respectivement, les rampes des parois 32 et des pattes 29, de telle sorte que les pattes 29 et 39 sont déplacées vers des positions radialement extérieures dans lesquelles elles libères respectivement les collerettes 27 et 45. Le ressort 8 peut alors se relâcher, ce qui provoque un recul simultané des pièces 5 et 6, et donc de l'aiguille 4, ainsi que du récipient 10 du fait du frottement du joint 25, vers une position de rétraction montrée sur la figure 8. Dans cette position, l'extrémité distale de l'aiguille 4 se trouve en deçà de la face distale de la pièce 7 et la collerette 45 se trouve en deçà, du côté proximal, des saillies 41.

Il apparaît de ce qui précède que l'invention apporte des améliorations déterminantes aux dispositifs homologues de la technique antérieure, en assurant une parfaite sécurité contre les risques de piqûres accidentelles susceptibles de se produire après l'injection.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. En particulier, le piston peut comprendre une zone transperçable placée en regard de l'extrémité proximale de l'aiguille, cette extrémité proximale dépassant, du côté proximale de la pièce 5 qui le comporte.

## Revendications

1. Dispositif (1) d'injection d'un produit, notamment à usage médical, comprenant :
- un corps (2) recevant une aiguille creuse d'injection (4) et un récipient (10) contenant le produit à injecter ; l'aiguille (4) est reliée au corps (2) en étant mobile par rapport à celui-ci entre une position d'injection et une position de rétraction ;
- un poussoir (9) monté coulissant sur le corps (2) et déplaçable par rapport à celui-ci pour réaliser l'injection ; ledit récipient (10) étant fermé à une extrémité;
- des moyens (5 à 7 ; 28, 29) de maintien de l'aiguille (4), maintenant normalement l'aiguille (4) en position d'injection, qui peuvent être relâchés pour libérer le déplacement de l'aiguille (4) vers ladite position de rétraction ;
- un piston (11) engagé dans le récipient (10), ledit piston (11) étant déplaçable dans le récipient (10) lorsque le poussoir (9) est déplacé par rapport au corps (2), ledit piston (11) étant conformé pour, dans une première conformation du piston (11) ou position relative de ce piston (11) et de ce récipient (10), fermer le récipient (10) de manière à isoler le produit par rapport à l'extérieur de ce récipient (10),
**caractérisé en ce que** ledit piston (11) est conformé pour, dans une deuxième conformation du piston (11) ou position relative de ce piston (11) et de ce récipient (10), permettre le passage du produit vers l'extérieur du récipient (10),
le dispositif (1) d'injection comprenant en outre :
- des moyens (45, 39, 41) de maintien du récipient (10) reliant ledit récipient (10) audit poussoir (9), ledit récipient (10) étant mobile par rapport au poussoir (9) entre une position permettant l'injection et une position de rétraction, lesdits moyens (45, 39, 41) de maintien du récipient (10) maintenant normalement le récipient (10) en position permettant l'injection, et pouvant être relâchés pour libérer le déplacement du récipient (10) vers ladite position de rétraction ;
- des moyens respectifs (32, 42) d'actionnement desdits moyens (5 à 7 ; 28, 29) de maintien de l'aiguille (4) et desdits moyens (45, 39, 41) de maintien du récipient (10), lesdits moyens respectifs d'actionnement étant aptes, en fin d'injection, à relâcher les moyens de maintien de l'aiguille (4) avant le relâchement des moyens de maintien du récipient (10), ou simultanément à ce relâchement.

2. Dispositif (1) d'injection selon la revendication 1, **caractérisé en ce que** le piston (11) est conformé pour, dans ladite deuxième conformation ou position, permettre le passage du produit entre lui et le récipient (10).

3. Dispositif (1) d'injection selon la revendication 2, **caractérisé en ce que** le piston (11) comprend au moins une zone périphérique, propre, dans ladite première conformation du piston (11), à appuyer étroitement contre la paroi du récipient (10), et, dans ladite deuxième conformation du piston (11), à s'effacer sous la pression du produit à injecter pour permettre le passage de ce dernier.

4. Dispositif (1) d'injection selon la revendication 1, **caractérisé en ce que** le piston comprend une zone transperçable placée en regard de l'extrémité proximale de l'aiguille.

5. Dispositif (1) d'injection selon l'une des revendications 1 à 4, **caractérisé en ce qu'**il comprend des moyens (8) à ressort permettant d'amener l'aiguille (4) et le récipient (10) en position de rétraction sans intervention volontaire extérieure.

6. Dispositif (1) d'injection selon l'une des revendications 1 à 5, **caractérisé en ce que** ledit corps (2) forme une paroi distale (7, 21) perpendiculaire à l'axe de l'aiguille (4), de laquelle l'aiguille (4) dépasse, en position d'injection, sur une longueur correspondant à la profondeur d'enfoncement recherchée de cette aiguille (4) lors de l'injection.

7. Dispositif (1) d'injection selon l'une des revendications 1 à 6, **caractérisé en ce que** lesdits moyens de maintien de l'aiguille (4) comprennent :
- une pièce (6) supportant l'aiguille (4), comportant au moins un moyen de verrouillage (27) ;
- au moins une patte (29) comportant un moyen de verrouillage (28) propre à coopérer avec celui de ladite pièce (6) supportant l'aiguille (4), cette patte (29) étant mobile radialement entre une position radialement interne normale, dans laquelle lesdits moyens de verrouillage (27, 29) viennent en prise de manière à maintenir ladite pièce (6) supportant l'aiguille (4) par rapport audit corps (2), et une position radialement externe, dans laquelle une zone (42) du poussoir (9) vient déplacer cette patte (29) radialement vers l'extérieur de manière à libérer ledit verrouillage, ce qui libère par conséquent ladite pièce (6) supportant l'aiguille (4) par rapport audit corps (2).

8. Dispositif (1) d'injection selon l'une des revendications 1 à 7, **caractérisé en ce que** lesdits moyens de maintien du récipient (10) comprennent :
- une collerette (45) formée au niveau de l'extrémité du récipient (10) opposée à l'extrémité fermée de ce récipient (10) ;
- des moyens de prise (41) solidaires dudit poussoir (9), permettant de relier ladite collerette (45) au poussoir (9) ; et
- au moins une patte (39) comportant lesdits moyens de prise (41), mobile dans le sens radial de ce poussoir (9), entre une position radialement interne, dans laquelle lesdits moyens de prise (41) relient ladite collerette (45) au poussoir (9), et une position radialement externe, dans laquelle lesdits moyens de prise (41) sont effacés radialement au delà cette collerette (45), qu'ils libèrent par conséquent.

## Patentansprüche

1. Injektionsvorrichtung (1) eines Produkts, insbesondere zum medizinischen Gebrauch, die umfasst:
- einen Körper (2), der eine hohle Injektionsnadel (4) aufnimmt und einen Behälter (10), der das zu injizierende Produkt enthält; wobei die Nadel (4) mit dem Körper (2) zwischen einer Injektionsstellung und einer eingezogenen Stellung im Verhältnis zu diesem bewegbar verbunden ist,
- einen Drücker (9), der gleitend auf dem Körper (2) montiert und im Verhältnis zu diesem verlagerbar ist, um die Injektion durchzuführen, wobei der Behälter (10) an einem Ende geschlossen ist,
- Haltemittel (5 bis 7; 28, 29) der Nadel (4), die die Nadel (4) normal in Injektionsstellung halten, die losgelassen werden können, um die Verlagerung der Nadel (4) in die eingezogene Stellung freizugeben,
- einen Kolben (11), der in den Behälter (10) eingeführt ist, wobei der Kolben (11) in dem Behälter (10) verlagerbar ist, wenn der Drücker (9) im Verhältnis zum Körper (2) verlagert wird, wobei der Kolben (11) ausgebildet ist, um in einer ersten Ausbildung des Kolbens (11) oder relativen Stellung dieses Kolbens (11) und dieses Behälters (10) den Behälter (10) derart zu verschließen, dass das Produkt im Verhältnis zur Außenwelt dieses Behälters (10) isoliert ist,
**dadurch gekennzeichnet, dass** der Kolben (11) ausgebildet ist, um in einer zweiten Ausbildung des Kolbens (11) oder relativen Stellung dieses Kolbens (11) und dieses Behälters (10) den Durchgang des Produkts nach außerhalb des Behälters (10) zu erlauben,
wobei die Injektionsvorrichtung (1) ferner umfasst:
- Haltemittel (45, 39, 41) des Behälters (10), die den Behälter (10) mit dem Drücker (9) verbinden, wobei der Behälter (10) im Verhältnis zum Drücker (9) zwischen einer Stellung, die die Injektion erlaubt, und einer eingezogenen Stellung bewegbar ist, wobei die Haltemittel (45, 39, 41) des Behälters (10) den Behälter (10) normal in einer Stellung halten, die die Injektion erlaubt, und losgelassen werden können, um die Verlagerung des Behälters (10) in die eingezogene Stellung freizugeben,
- jeweilige Betätigungsmittel (32, 42) der Haltemittel (5 bis 7; 28, 29) der Nadel (4) und der Haltemittel (45, 39, 41) des Behälters (10), wobei die jeweiligen Betätigungsmittel imstande sind, am Ende der Injektion die Haltemittel der Nadel (4) vor dem Loslassen der Haltemittel des Behälters (10) loszulassen oder gleichzeitig mit diesem loszulassen.

2. Injektionsvorrichtung (1) nach Anspruch 1, **dadurch gekennzeichnet, dass** der Kolben (11) ausgebildet ist, um in der zweiten Ausbildung oder Stellung den Durchgang des Produkts zwischen ihm und dem Behälter (10) zu erlauben.

3. Injektionsvorrichtung (1) nach Anspruch 2, **dadurch gekennzeichnet, dass** der Kolben (11) in der ersten Ausbildung des Kolbens (11) mindestens eine periphere Zone umfasst, die imstande ist, eng gegen die Wand des Behälters (10) zu drücken und sich in der zweiten Ausbildung des Kolbens (11) unter dem Druck des zu injizierenden Produkts zu entfernen, um den Durchgang desselben zu erlauben.

4. Injektionsvorrichtung (1) nach Anspruch 1, **dadurch gekennzeichnet, dass** der Kolben eine durchstoßbare Zone umfasst, die gegenüber dem proximalen Ende der Nadel angeordnet ist.

5. Injektionsvorrichtung (1) nach einem der Ansprüche 1 bis 4, **dadurch gekennzeichnet, dass** sie Federmittel (8) umfasst, die es erlauben, die Nadel (4) und den Behälter (10) ohne gezielten Eingriff von außen in die eingezogene Stellung zu führen.

6. Injektionsvorrichtung (1) nach einem der Ansprüche 1 bis 5, **dadurch gekennzeichnet, dass** der Körper (2) eine distale Wand (7, 21) im rechten Winkel zur Achse der Nadel (4) bildet, ab der die Nadel (4) bei der Injektion in Injektionsstellung über eine entsprechende Länge in der beabsichtigten Eindringtiefe dieser Nadel (4) übersteht.

7. Injektionsvorrichtung (1) nach einem der Ansprüche 1 bis 6, **dadurch gekennzeichnet, dass** die Haltemittel der Nadel (4) umfassen:
- ein Teil (6), das die Nadel (4) trägt, welches mindestens ein Verriegelungsmittel (27) aufweist,
- mindestens einen Fuß (29), der ein Verriegelungsmittel (28) aufweist, das imstande ist, mit dem des Teils (6), das die Nadel (4) trägt, zusammenzuarbeiten, wobei dieser Fuß (29) zwischen einer radial internen normalen Stellung, in der die Verriegelungsmittel (27, 29) derart in Eingriff kommen, dass das Teil (6), das die Nadel (4) trägt, im Verhältnis zum Körper (2) gehalten wird, und einer radial externen Stellung, in der eine Zone (42) des Drückers (9) diesen Fuß (29) radial nach außen derart verlagert, dass die Verriegelung freigegeben wird, radial bewegbar ist, wodurch folglich das Teil (6), das die Nadel (4) trägt, im Verhältnis zum Körper (2) freigegeben wird.

8. Injektionsvorrichtung (1) nach einem der Ansprüche 1 bis 7, **dadurch gekennzeichnet, dass** die Haltemittel des Behälters (10) umfassen:
- einen Kragen (45), der auf Ebene des Endes des Behälters (10) ausgebildet ist, das dem geschlossenen Ende dieses Behälters (10) gegenüberliegt,
- Erfassungsmittel (41), die mit dem Drücker (9) verbunden sind, die es erlauben, den Kragen (45) mit dem Drücker (9) zu verbinden, und
- mindestens einen Fuß (39), der die Erfassungsmittel (41) aufweist, der in die radiale Richtung dieses Drücker (9) zwischen einer radial internen Stellung, in der die Erfassungsmittel (41) den Kragen (45) mit dem Drücker (9) verbinden, und einer radial externen Stellung, in der die Eingriffmittel (41) radial jenseits dieses Kragens (45) beabstandet sind, den sie folglich freigeben, bewegbar ist.

## Claims

1. A device (1) for injecting a product, particularly for medical use, which comprises:
- a body (2) housing a hollow injection needle (4) and a container (10) containing the injectable product; the needle (4) is connected to the body (2) but able to move relative to the latter between an injection position and a retracted position;
- a plunger (9) that slides in the body (2) and is displaceable relative to the latter to perform the injection; said container (10) being closed at one end;
- means (5-7; 28, 29) for keeping the needle (4) in position, which means normally keep the needle (4) in the injection position and can be released to free the needle (4) to move to said retracted position;
- a piston (11) engaged in the container (10), said piston (11) being movable in the container (10) when the plunger (9) is moved with respect to the body (2), said piston (1) being so shaped that, in a first configuration of the piston or relative position of this piston (11) and of this container (10), it closes the container (10) in such a way as to isolate the product from the environment outside this container (10),
**characterized in that** said piston (11) is so shaped that, in a second configuration of the piston (11) or relative position of this piston (11) and of this container (10), it allows the product to pass out of the container (10),
the device further comprising
- means (45, 39, 41) for keeping the container (10) in position and linking said container (10) to said plunger (9), said container (10) being movable with respect to the plunger (9) between a position that enables the injection to be performed and a retraction position, said means (45, 39, 41) for keeping the container (10) in position normally keeping the container (10) in the position that enables the injection to be performed, and being releasable to free the container (10) to move to said retracted position;
- respective means (32, 42) for operating said means (5-7; 28, 29) for keeping the needle (4) in position and said means (45, 39, 41) for keeping the container (10) in position, which, at the end of the injection, release the means for keeping the needle (4) in position before, or at the same time as, the means for keeping the container (10) in position are released.

2. The injection device (1) as claimed in claim 1, in which the piston (11) is so shaped that, in said second configuration or position, it allows the product to pass between itself and the container.

3. The injection device (1) as claimed in claim 2, in which the piston (11) comprises at least one peripheral zone that is able, in said first configuration of the piston (11), to press tightly against the wall of the container (10), and, in said second configuration of the piston (11), to withdraw under the pressure of the injectable product to allow the latter to pass it.

4. The injection device (1) as claimed in claim 1, in which the piston comprises a pierceable zone located in line with the proximal end of the needle.

5. The injection device (1) as claimed in one of claims 1-4, which comprises spring means (8) for moving the needle (4) and the container (10) to the retracted position without voluntary external action.

6. The injection device (1) as claimed in one of claims 1-5, in which said body (2) forms a distal wall (7, 21) perpendicular to the axis of the needle (4), from which the needle (4) projects, in the injection position, to a distance equal to the desired depth of insertion of this needle (4) during the injection.

7. The injection device (1) as claimed in one of claims 1-6, in which said means for keeping the needle (4) in position comprise:
- a needle-supporting part (6) comprising at least one locking means (27); and
- at least one tab (29) that comprises a locking means (28) able to engage with that of said needle-supporting part (6), this tab (29) being moveable radially between a normal, radially inward position, in which said locking means (27, 29) engage with each other to keep said needle-supporting part (6) in position relative to said body (2), and a radially outward position, in which a zone (42) of the plunger (9) moves this tab (29) radially out to unlock it, thereby freeing said needle-supporting part (6) from said body (2).

8. The injection device (1) as claimed in one of claims 1-7, in which said means for keeping the container (10) in position comprise:
- a flange (45) formed at the opposite end of the container (10) from the closed end of this container (10);
- engagement means (41) integral with said plunger (9) for connecting said flange (45) to the plunger (9) ; and
- at least one tab (39) comprising said engagement means (41) and able to move in the radial direction of this plunger (9) between a radially inward position, in which said engagement means (41) connect said flange (45) to the plunger (9), and a radially outward position, in which said engagement means (41) are withdrawn radially wide of this flange (45), thereby releasing it.
